# EUROPÄISCHE PATENTANMELDUNG

(11) **EP 2 055 694 A1**
(43) Veröffentlichungstag der Anmeldung: **06.05.2009**
(21) Anmeldenummer: 07021104.0
(22) Anmeldetag: 29.10.2007
(51) Int. Cl.: C07C 67/327, C07C 67/46, C07C 69/734, C07C 69/708

(54) **Verfahren zur Herstellung von Alkyl-3-alkoxyprop-2-enoaten**

(71) Anmelder: Lonza AG, 4052 Basel (CH)
(72) Erfinder: Zur Täschler, Cornelia, 3912 Termen (CH); Zollinger, Daniel, 3700 Spiez (CH); Wenger, Wolfgang, 3930 Visp (CH)

(57) **Zusammenfassung**

Die vorliegende Erfindung betrifft ein Verfahren zur Herstellung von Alkyl-3-alkoxyprop-2-enoaten der Formel ROCH=CHCO₂R, wobei R C₁₋₆ Alkyl bedeutet, aus den entsprechenden Alkyl-3,3-dialkoxypropionaten der Formel (RO)₂CHCH₂CO₂R durch Eliminierung des entsprechenden Alkohols (ROH) unter Wärmezufuhr und in Gegenwart von Methansulfonsäure.

## Beschreibung

Die vorliegende Erfindung betrifft ein Verfahren zur Herstellung von Alkyl-3-alkoxyprop-2-enoaten (ROCH=CHCO₂R aus Alkyl-3,3-dialkoxypropionaten ((RO)₂CHCH₂CO₂R), wobei R C₁₋₆ Alkyl bedeutet.

Alkyl-3-alkoxyprop-2-enoate sind wichtige C-3 Bausteine und werden beispielsweise für die Herstellung von Alkyl-2,2,3-trichlor-3-alkoxypropionaten, Pyrazolen, Furanonen, Thiophenen, Aminothiazolen, Isoxazol und Vitamin A verwendet.

Es ist bekannt, dass Alkyl-3-alkoxyprop-2-enoate aus Alkyl-3,3-dialkoxypropionaten durch säurekatalysierte Eliminierung von Alkohol hergestellt werden können. So verwendet beispielsweise US 2,571,212 Natriumhydrogensulfat oder p-Toluolsulfonsäure als Katalysatoren. Diese Substanzen sind zwar chemisch sehr effektiv, haben aber bei der Reaktionsführung mehrere Nachteile, vor allem in grosstechnischer Produktion. Die nach der Alkoholabspaltung gebildeten Alkyl-3-alkoxyprop-2-enoate werden zweckmässig durch Destillation oder Rektifikation aufgearbeitet. Da Natriumhydrogensulfat und *p*-Toluolsulfonsäure bei Standardbedingungen Feststoffe sind, wirkt sich dies vor allem gegen Ende der Destillation negativ aus. So bilden sich im Destillationssumpf unerwünschte Blasen, die Schwankungen bei der Destillation hervorrufen. Ausserdem wird nach Beendigung der Destillation der Sumpfrückstand fest, so dass die nachfolgende Reinigung und Entsorgung schwierig ist. Aufgabe der vorliegenden Erfindung war es deshalb, einen Katalysator zu finden, der bei vergleichbarer chemischer Effektivität diese Nachteile nicht aufweist, und so eine einfache Handhabung, vor allem bei Produktion im kommerziellen Massstab, ermöglicht.

Erfindungsgemäss wird diese Aufgabe durch das Verfahren nach Anspruch 1 gelöst.

Beansprucht wird ein Verfahren zur Herstellung von Alkyl-3-alkoxyprop-2-enoaten der Formel ROCH=CHCO₂R, wobei R C₁₋₆ Alkyl bedeutet, aus den entspechenden Alkyl-3,3-dialkoxy-propionaten der Formel (RO)₂CHCH₂CO₂R durch säurekatalysierte Eliminierung von einem Molekül des entsprechenden Alkohols (ROH) unter Wärmezufuhr, dadurch gekennzeichnet, dass Methansulfonsäure als saurer Katalysator eingesetzt wird.

Unter dem Begriff "C₁₋₆ Alkyl" ist hier und im Folgenden jede lineare oder verzweigte Alkylgruppe, die 1 bis 6 Kohlenstoffatome enthält, zu verstehen. Beispiele für C₁₋₆ Alkyl sind Methyl, Ethyl, Propyl, Isopropyl, Butyl, Isobutyl, *sec*-Butyl, *tert*-Butyl, Pentyl, Isopentyl (3-Methylbutyl), Neopentyl (2,2-Dimethylpropyl), Hexyl, Isohexyl (4-Methylpentyl) und ähnliche.

Das eingesetzte Alkyl-3,3-dialkoxypropionat kann auf bekannte Weise, wie beispielsweise in US 2,449,471 beschrieben, oder gemäss dem unten dargestellten Verfahren hergestellt werden.

Vorzugsweise beträgt der Anteil an Methansulfonsäure zwischen 0,05 Gewichts-% und 15 Gewichts-% (bezogen auf Alkyl-3,3-dialkoxypropionat), besonders bevorzugt zwischen 0,1 Gewichts-% und 10 Gewichts-%. Die Methansulfonsäure kann an geeignete Trägermaterialien, wie etwa Kieselgel, gebunden sein.

Als Lösungsmittel kann jedes Lösungsmittel eingesetzt werden, das nicht mit den Reaktionskomponenten reagiert, wie beispielsweise Ligroin. Die Eliminierung wird vorzugsweise ohne Lösungsmittel durchgeführt.

Die Eliminierung wird bevorzugt bei einer Temperatur zwischen 50 °C und 250 °C, besonders bevorzugt zwischen 80 °C und 200 °C, durchgeführt, und die Reaktionsdauer liegt vorteilhaft zwischen 1 Stunde und 15 Stunden, bevorzugt zwischen 1 Stunde und 10 Stunden. Gegebenenfalls, kann die Reaktion auch bei reduziertem Druck durchgeführt werden. Bei der Eliminierung bildet sich bevorzugt das *E*-Isomere des Alkyl-3-alkoxyprop-2-enoats. Zweckmässig wird der gebildete Alkohol (ROH) während der Reaktion direkt abdestilliert.

Nach der Eliminierung kann das erhalte Alkyl-3-alkoxyprop-2-enoat in bekannter Weise, beispielsweise durch Rektifikation, gereinigt werden.

Ein weiterer Aspekt der vorliegenden Erfindung ist ein kontinuierliches Verfahren zur Herstellung der im erfindungsgemässen obigen Verfahren eingesetzten Alkyl-3,3-dialkoxypropionate ((RO)₂CHCH₂CO₂R), wobei R wie oben definiert ist.

Eine Herstellungsmöglichkeit für Alkyl-3,3-dialkoxypropionate ist die Herstellung aus dem entsprechenden Orthoameisensäureester durch Reaktion mit Keten in Gegenwart eines sauren Katalysators. So stellt zum Beispiel G. Büchi das Methyl-3,3-dimethoxypropionat in 19 % iger Ausbeute bei einer Reaktionstemperatur von -70 °C her (Büchi et al., J. Am. Chem. Soc. 1973, 95, 540-545). Die Herstellung von Ethyl-3,3-diethoxypropionat ist beispielsweise in US 2,449,471 mit einer Ausbeute von 52 % und in D. Crosby et al., J. Org Chem. 1962, 27, 3083-3085 mit einer Ausbeute von 54 % beschrieben. In den hier zitierten Literaturstellen wurde als saurer Katalysator das Addukt aus Bortrifluorid und Diethylether verwendet und die Reaktanten diskontinuierlich zur Reaktion gebracht. Da es sich um eine stark exotherme Reaktion handelt und Keten in grossen Mengen schwierig zu handhaben ist, erlaubt die diskontinuierliche Reaktion nur Ansätze im Labormassstab.

Die Herstellung von Alkyl-3,3-dialkoxypropionaten der Formel (RO)₂CHCH₂CO₂R, wobei R wie oben definiert ist, erfolgt vorzugsweise durch Reaktion von Keten (CH₂=C=O) mit dem entsprechenden Orthoameisensäureester ((RO)₃CH) in Gegenwart eines sauren Katalysators, wobei die Reaktion kontinuierlich in einem Schlaufenreaktor durchgeführt wird.

In einer bevorzugten Ausführungsform ist der Orthoameisensäureester ausgewählt aus der Gruppe bestehend aus Trimethylorthoformiat, Triethylorthoformiat, Tripropylorthoformiat und Tributylorthoformiat. Besonders bevorzugt ist Trimethylorthoformiat.

"Kontinuierlicher Betrieb" bedeutet, dass sowohl die Reaktionspartner als auch die Reaktionsprodukte kontinuierlich zugegeben bzw. entfernt werden. Erfindungsgemäss werden dabei das Ketengas, der Orthoameisensäurester und der saure Katalysator kontinuierlich in einem Schlaufenreaktor miteinander zur Reaktion gebracht.

Der Begriff "Schlaufenreaktor" steht hierbei nicht für eine bestimmte Bauform, sondern lediglich für das Funktionsprinzip. Im einfachsten Fall besteht der Schlaufenreaktor aus einem ringförmig geschlossenen Rohr (Schlaufe), in das eine Umwälzpumpe eingebaut ist. An der Schlaufe befindet sich mindestens ein Anschluss zum Abziehen des Reaktionsgemisches und mindestens zwei Anschlüsse zum Einspeisen der Reaktionskomponenten.
Die Reaktion kann in einem Lösungsmittel oder ohne Anwesenheit eines Lösungsmittels durchgeführt werden und der saure Katalysator kann direkt in den Reaktor gegeben oder zuvor mit dem Orthoameisensäureester und/oder mit dem Lösungsmittel vermischt werden. Die Anzahl und Lage der Einspeisungsanschlüsse sind entsprechend zu wählen. Bevorzugt wird der Orthoameisensäureester vorgängig mit dem sauren Katalysator und gegebenenfalls mit dem Lösungsmittel vermischt, wobei sich der Katalysator löst oder nur suspendiert wird, und dann in den Schlaulenreaktor eingespeist. In einer besonders bevorzugten Verfahrensvariante wird die Reaktion ohne Anwesenheit eines Lösungsmittels durchgeführt.

Das Ketengas kann durch jede geeignete Methode in die Reaktionsmischung eingespeist werden, wie beispielsweise mittels Einleitungsrohr, das gegebenenfalls mit einer Düse oder Fritte versehen ist. Besonders bevorzugt ist eine Anordnung bei der ein Ketenstrahl und ein Strahl bestehend aus Orthoameisensäureestcr und Katalysator derart eingeleitet werden, dass letzterer das Gas erfassen und zerteilen kann, was zum Beispiel in einem Schlaufenreaktor mit Treibstrahlantrieb verwirklicht ist. Dadurch wird das Gas besser in der Flüssigkeit dispergiert. In einer besonders bevorzugten Ausführungsform findet die Reaktion in einem Strahldüsenreaktor (Jet-Reaktor) statt. Die Strahldüsenfunktion bewirkt, dass das Keten im Mischrohr fein dispergiert wird, was zu einem optimalen Wirkungsgrad der nachfolgenden chemischen Reaktion führt. Ausserdem erlaubt diese Anordnung ein gleichmässiges, druckfreies Nachfliessen von Keten, was besonders erwünscht ist, da Keten unter Druckeinwirkung zur Polymerisation neigt.

Die Einspeisung der Reaktionskomponenten in den Schlaufenreaktor erfolgt im Wesentlichen simultan und kontinuierlich. Dies bedeutet, dass keine grösseren Unterbrechungen oder starke Schwankungen des molaren Verhältnisses der Reaktanten im Reaktionsgemisch auftreten. Die Zirkulation in der Schlaufe bewirkt eine gute oder gar ideale Vermischung, wobei es jedoch nicht notwendig ist, eine ideale Vermischung zu erzwingen.

Gleichzeitig mit der Einspeisung der Reaktionskomponenten wird ein, dem eingespeisten Volumen entsprechender Strom des Reaktionsgemisches aus dem Schlaufenreaktor abgezogen und der Aufbereitung zugeführt. Dies kann zum Beispiel durch einen einfachen Überlauf oder durch Abpumpen geschehen, wobei das Abpumpen mit Hilfe einer Niveausonde geregelt werden kann.

Die wegen der starken Exothermie der Reaktion erforderliche Kühlung kann mit bekannten Mitteln, wie etwa durch einen, sich über einen wesentlichen Teil der Rohrlänge erstreckenden Kühlmantel oder durch einen in der Schlaufe eingebauten Wärmeaustauscher üblicher Bauart erreicht werden.

Die Reaktion wird vorteilhaft bei einer Temperatur zwischen -40 °C und 50 °C durchgeführt, wobei die eingespeisten Reaktionskomponenten vorgekühlt eingespeist werden können. Bevorzugt ist eine Reaktionstemperatur zwischen -30 °C und 30 °C, und besonders bevorzugt zwischen -10 °C und 10°C.

Das verwendete Keten kann rein sein oder Inertgase wie Stickstoff, Kohlenmonoxid und Kohlendioxid enthalten, die zweckmässig mittels einer geeigneten Entlüftungsvorrichtung aus dem Schlaufenreaktor entfernt werden, um einen Überdruck zu vermeiden.

Das molare Verhältnis von Orhoameisensäureester zu Keten beträgt bevorzugt 0,9 bis 1,2. Besonders bevorzugt ist ein molares Verhältnis von 1,0 bis 1,1. Diese Werte beziehen sich auf die eingespeisten Mengen. Die im Reaktionsgemisch tatsächlich vorliegenden Verhältnisse können sich davon mehr oder weniger stark unterscheiden.

Als Lösungsmittel kann grundsätzlich jedes organische Lösungsmittel eingesetzt werden, in dem der Orthoameisensäureester genügend löslich ist und das nicht mit Keten oder einer anderen Komponente reagiert. Geeignete Lösungsmittel sind beispielsweise aliphatische oder aromatische Kohlenwasserstoffe und Ether. Es ist aber auch möglich, auf den Einsatz eines Lösungsmittels zu verzichten, falls der eingesetzte Orthoameisensäureester flüssig ist. In einer bevorzugten Verfahrensvariante wird Trimethylorthoformiat ohne Lösungsmittel direkt mit Keten unter Anwesenheit eines sauren Katalysators zur Reaktion gebracht.

Die Reaktion kann durch jeden geeigneten sauren Katalysator katalysiert werden. Als saure Katalysatoren eignen sich sowohl "klassische" *Lewis*-Säuren, als auch "klassische" *Brönsted-*Säuren, aber auch saure Polysilikate. Zweckmässig werden als "klassische" Lewis-Säuren Zink(II)-chlorid, Eisen(III)-chlorid, Aluminiumchlorid, Bortrifluorid und seine Addukte mit Ethern, Estern und ähnlichen Verbindungen eingesetzt. Ein bevorzugtes Addukt des Bortrifluorids ist das Addukt mit Diethylether. Bevorzugte Beispiele für "klassische" *Brönsted-*Säuren sind Schwefelsäure, Phosphorsäure, Methansulfonsäure und Benzolsulfonsäure.
Saure Polysilikate haben *Lewis-* und/oder *Brönsied*-Säureeigenschaften und sind daher für das erfindungsgemässe Verfahren ebenfalls geeignet. Die sauren Polysilikate können auch modifiziert oder in Mischungen eingesetzt werden. Die nachfolgend aufgeführten Formeln sind hier lediglich zur Verdeutlichung der Polysilikate angegeben und sind keinesfalls als Einschränkung aufzufassen. Geeignete saure Polysilikate sind beispielsweise amorphe Polysilikate des Allophan-Typs; kettenförmige Polysilikate des Hormit-Typs, wie etwa das "Polygorskit"; zweischichtige Polysilikate des Kaolin-Typs, wie etwa "Kaolinit" Al₂(OH)₄(Si₂O₅] und "Halloysit" Al₂(OH)₄[Si₂O₅] × 2 H₂O; dreischichtige Polysilikate des Smectit-Typs, wie etwa "Sauconit" Na_{0,3}Zn₃(Si,Al)₄O₁₀(OH)₂ × 4 H₂O, "Saponit" (Ca₂,Na)_{0,3}(Mg,Fe²⁺)₃(Si,Al)₄O₁₀(OH)₂ × 4 H₂O, "Montmorillonit" M⁺_{0,3}(Al,Mg)₂Si₄O₁₀(OH)₂ × *n* H₂O, wobei M⁺ in natürlichem Montmorillonit eines oder mehrere der Kationen Na⁺, K⁺, Mg²⁺ und Ca²⁺ bedeutet, "Vermiculit" (Mg,Fe²⁺,Al)₃(Al,Si)₄O₁₀(OH)₂ × 4 H₂O, "Nontronit" Na_{0,3}Fe₂³⁺(Si,Al)₄O₁₀(OH)₂ × 4 H₂O und "Hectorit" Na_{0,3}(Mg,Li)₃Si₄O₁₀(F,OH)₂; dreischichtige Polysilikate des Illit-Typs; variabelschichtige Polysilikate des Chlorit-Typs und Gerüstpolysilikate, wie etwa Zeolithe, bevorzugt des Typs Y in seiner H-Form. Die sauren Polysilikate des erfindungsgemässen Verfahrens können bei Bedarf durch Behandlung mit Säure und/oder durch Behandlung mit einer Metallsalzlösung und/oder durch Trocknung, und im Falle von Zeolithen bevorzugt durch Ionenaustausch und/oder durch Erhitzen, aktiviert werden. In einer bevorzugten Ausführungsform werden als Katalysatoren saure Polysilikate des Smectit-Typs und Zeolithe verwendet. Ein besonders bevorzugtes saures Polysilikat des Smectit-Typs ist das Montmorillonit und hier vor allem die unter den Bezeichnungen "Montmorillonit K 10" und "Montmorillonit KSF/0" beispielsweise von der Firma Süd-Chemie erhältlichen Typen.

Der saure Katalysator wird vorteilhaft in einer Menge zwischen 0,1 Gewichts-% und 20 Gewichts-% (bezogen auf Orthoameisensäureester) eingesetzt, bevorzugt zwischen 0,5 und 10 Gewichts-%. Die Menge ist allerdings von der Aktivität des Katalysators und der Reaktionstemperatur abhängig.

Bei der Reaktionsführung muss darauf geachtet werden, dass der Gehalt an Wasser so gering wie möglich ist, da Keten und Orthoameisensäureester unerwünscht mit Wasser reagieren.

Die Aufarbeitung erfolgt nach an sich bekannten Methoden und hängt im Wesentlichen von den physikalischen Eigenschaften des gebildeten Alkyl-3,3-dialkoxypropionats und der übrigen Komponenten der Reaktionsmischung ab. Bei Verwendung eines festen Säurekatalysators wird dieser vorteilhaft abfiltriert und das Filtrat aufgearbeitet, während bei Verwendung eines flüssigen Säurekatalysators dieser zunächst in der Reaktionsmischung neutralisiert wird. Die Neutralisation kann beispielsweise durch Zugabe von basischen Alkalimetallsalzen wie Natriumhydroxid und Kaliumcarbonat oder durch Zugabe von Alkalimetallalkoholaten wie Natriummethanolat und Kaliumethanolat oder durch Zugabe von ähnlich basischen Reagenzien, wie etwa wasserfreiem Ammoniak, erfolgen. Ausfallender Katalysator kann dann abfiltriert und das Filtrat bei Bedarf anschliessend weiter gereinigt werden.
In einer bevorzugten Ausführungsform wird ein fester Säurekatalysator verwendet, der in einem ersten Aufarbeitungsschritt abfiltriert wird. Entweder wird dieser abfiltrierte Rückstand verworfen oder, bei Bedarf nach Aufreinigung und gegebenenfalls Reaktivierung, wieder als saurer Katalysator in der Reaktionsmischung eingesetzt.

Nach Entfernen des sauren Katalysators wird das Filtrat auf bekannte Weise, bevorzugt durch Destillation, aufgearbeitet, um das gebildete Alkyl-3,3-dialkoxypropionat in reiner Form zu erhalten. In einer besonders bevorzugten Ausführungsform wird der gewöhnlich tiefer als das gewünschte Produkt siedende, nicht umgesetzte Orthoameisensäureester nach der Filtration abdestilliert und der Reaktionsmischung wieder zugeführt, was den Gesamtumsatz der Reaktion deutlich erhöht.

### Erläuterung der Zeichnung

Die beigefügte Schemazeichnung dient ebenso wie die Ausführungsbeispiele lediglich zur Veranschaulichung des Erfindungsgegenstands, ohne diesen auf die gezeigten Ausführungsformen zu beschränken.

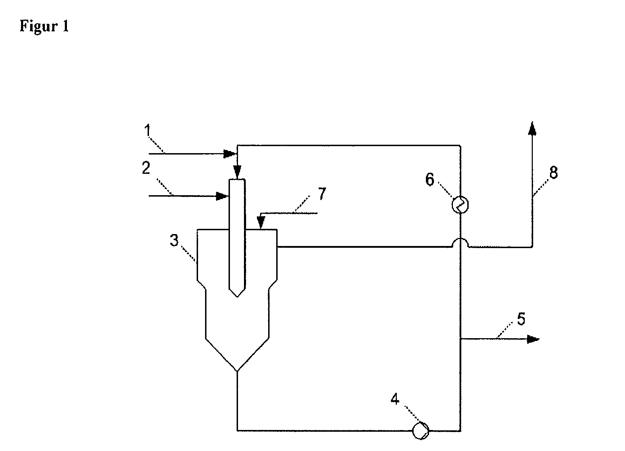

Zeichnung 1 zeigt schematisch eine Vorrichtung zur kontinuierlichen Herstellung von Alkyl-3,3-dialkoxypropionat. Die Bezugszeichen bedeuten im Einzelnen:
1. Einspeisung der Mischung aus Orthoester und saurem Katalysator
2. Keteneinspeisung
3. Strahldüsenreaktor
4. Umwälzpumpe
5. Produktentnahme
6. Wärmeaustauscher
7. Stickstoffzuführung
8. Entlüftung von Inertgasen

### Beispiele

Die folgenden Beispiele verdeutlichen die Ausführung der Erfindung, ohne dass darin eine Einschränkung zu sehen ist.

### Beispiel 1: Herstellung von Methyl-3,3-dimethoxypropionat

In einen auf 0 °C Innentemperatur gekühlten, inertisierten 620 L Strahldüsenreaktor (siehe Zeichnung 1) wurden gleichzeitig, aber getrennt voneinander 150 kg/h (1,413 kmol/h) Trimethylorthoformiat (Fluka) enthaltend 1,5 Gewichts-% Montmorillonit K 10 (Süd-Chemie) und 84 kg/h Keten (Gehalt an Keten ca. 70 %, Rest inerte Gase wie N₂, CO und CO₂, d.h. reines Keten ca. 59 kg/h, also ca. 1,4 kmol/h) eingespeist. Unter Stickstoffatmosphäre wurde das Reaktionsgemisch auf einer Temperatur von ca. 0 °C gehalten und über eine Umwälzpumpe in der Schlaufe zirkuliert. Entsprechend der Menge an zugegebenen Edukten lief ebenfalls kontinuierlich ein entsprechender Teil der Reaktionsmischung in einen Sammeltank über. Nach Filtration wurde die Reinheit des Filtrats mittels GC zu 80 % Methyl-3,3-dimethoxypropionat, 8 % nicht reagiertes Trimethylorthoformiat, 4 % Methyl-3-methoxyprop-2-enoat und 4 % Methylacetat bestimmt.
Aufgrund seines niedrigen Siedepunktes konnte Trimethylorthoformiat leicht abdestilliert werden. Das so zurückgewonnene Edukt wurde anschliessend der Reaktionsmischung wieder zugeführt.
Die Ausbeute des Methyl-3,3-dimethoxypropionats war 82 % (bezogen auf den Umsatz).

### Beispiel 2: Herstellung von Methyl-3-methoxyprop-2-enoat

In eine Destillationsapparatur mit Rundkolben wurden unter Stickstoffatmosphäre 0,2 g (2 mmol) Methansulfonsäure (Fluka) zu 150 g eines, analog Beispiel 1 hergestellten Filtrats (ca. 85 %, 0,86 mol Methyl-3,3-dimethoxypropionat) gegeben. Die Mischung wurde unter ständigem Einleiten von Stickstoff allmählich auf 160 °C erhitzt, wobei das gebildete Methanol direkt abdestilliert wurde. Nach sechs Stunden wurde die Hitzezufuhr abgebrochen. Das so erhaltene Methyl-3-methoxyprop-2-enoat war 88 % rein (GC) und wurde mittels Rektifikation bei 10 kPa gereinigt. Die Ausbeute betrug 85 g (85 %) Methyl-3-methoxyprop-2-enoat (K_{10 kPa} = 95 °C) mit einer Reinheit von 99 % (GC).

### Beispiel 3: Herstellung von Methyl-3-methoxyprop-2-enoat

Nach destillativem Entfernen des nicht umgesetzten Trimethylorthoformiats aus Beispiel 1 wurden 4,4 t (30 kmol) des so erhaltenen Methyl-3,3-dimethoxypropionats unter Stickstoffatmosphäre mit 6 kg (62 mol) Methansulfonsäure analog Beispiel 2 zur Reaktion gebracht. Nach Rektifikation bei 10 kPa erhielt man 2,4 t (21 kmol, 69 % bezogen auf eingesetztes Trimethylorthoformiat) Methyl-3-methoxyprop-2-enoat (K_{10 kPa} = 95 °C) in 93 % iger Reinheit (GC).

## Patentansprüche

1. Verfahren zur Herstellung von Alkyl-3-alkoxyprop-2-enoaten der Formel ROCH=CHCO₂R, wobei R C₁₋₆ Alkyl bedeutet, aus den entspechenden Alkyl-3,3-dialkoxypropionaten der Formel (RO)₂CHCH₂CO₂R durch säurekatalysierte Eliminierung des entsprechenden Alkohols (ROH) unter Wärmezufuhr, **dadurch gekennzeichnet, dass** Methansulfonsäure als saurer Katalysator eingesetzt wird.

2. Verfahren nach Anspruch 1, **dadurch gekennzeichnet, dass** die Methansulfonsäure in einer Menge zwischen 0,05 Gewichts-% und 15 Gewichts-% (bezogen auf Alkyl-3,3-dialkoxypropionat) eingesetzt wird.

3. Verfahren nach Anspruch 1 oder 2, **dadurch gekennzeichnet, dass** das Verfahren ohne Anwesenheit eines Lösungsmittels durchgeführt wird.

4. Verfahren nach einem der Ansprüche 1 bis 3, **dadurch gekennzeichnet, dass** die Reaktion bei einer Temperatur zwischen 50 °C und 250 °C durchgeführt wird.

5. Verfahren nach einem der Ansprüche 1 bis 4, **dadurch gekennzeichnet, dass** die Reaktionsdauer zwischen 1 Stunde und 15 Stunden beträgt.

6. Verfahren nach einem der vorhergehenden Ansprüche, **dadurch gekennzeichnet, dass** das eingesetzte Alkyl-3,3-dialkoxypropionat der Formel (RO)₂CHCH₂CO₂R, wobei R wie oben definiert ist, durch Reaktion von Keten mit dem entsprechenden Orthoameisensäureester der Formel (RO)₃CH in Gegenwart eines sauren Katalysators kontinuierlich in einem Schlaufenreaktor hergestellt wird.

7. Verfahren nach Anspruch 6, **dadurch gekennzeichnet, dass** der Orthoameisensäureester ausgewählt ist aus der Gruppe bestehend aus Trimethylorthoformiat, Triethylorthoformiat, Tripropylorthoformiat und Tributylorthoformiat.

8. Verfahren nach Anspruch 6 oder 7, **dadurch gekennzeichnet, dass** der Orthoameisensäureester vorgängig mit dem sauren Katalysator gemischt und erst dann in den Schlaufenreaktor eingespeist wird.

9. Verfahren nach einem der Ansprüche 6 bis 8, **dadurch gekennzeichnet, dass** der Schlaufenreaktor ein Strahldüsenreaktor ist.

10. Verfahren nach einem der Ansprüche 6 bis 9, **dadurch gekennzeichnet, dass** das molare Verhältnis von Orthoameisensäureester zu Keten 0,9 bis 1,2 beträgt.

11. Verfahren nach einem der Ansprüche 6 bis 10, **dadurch gekennzeichnet, dass** das Verfahren ohne Anwesenheit eines Lösungsmittels durchgeführt wird.

12. Verfahren nach einem der Ansprüche 6 bis 11, **dadurch gekennzeichnet, dass** der Orthoameisensäureester Trimethylorthoformiat und der saure Katalysator Montmorillonit ist.
